(19) Europäisches Patentamt
European Patent Office
Office européen des brevets

(11) **EP 2 460 526 A2**

(12) **EUROPEAN PATENT APPLICATION**
published in accordance with Art. 153(4) EPC

(43) Date of publication:
**06.06.2012 Bulletin 2012/23**

(21) Application number: **10804692.1**

(22) Date of filing: **27.07.2010**

(51) Int Cl.:
*A61K 35/64* (2006.01)    *A61P 17/00* (2006.01)
*A61K 8/98* (2006.01)    *A61Q 19/10* (2006.01)

(86) International application number:
**PCT/KR2010/004912**

(87) International publication number:
**WO 2011/013979 (03.02.2011 Gazette 2011/05)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **28.07.2009 KR 20090068925**

(71) Applicants:
• **Republic Of Korea**
**(Management : Rural Development Administration)**
**Gyeonggi-do 441-707 (KR)**
• **Dong Sung Pharm. Co., Ltd.**
**Dobong-gu**
**Seoul 132-020 (KR)**

(72) Inventors:
• **HAN, Sang Mi**
**Seosan-si**
**Chungcheongnam-do 356-882 (KR)**

• **LEE, Kwang Gil**
**Suwon-si**
**Gyeonggi-do 441-759 (KR)**
• **YEO, Ju Hong**
**Suwon-si**
**Gyeonggi-do 443-709 (KR)**
• **LEE, Yang Gu**
**Seoul 132-020 (KR)**
• **HAN, Chung Sub**
**Cheonan-si**
**Chungcheongnam-do 331-778 (KR)**

(74) Representative: **Pfenning, Meinig & Partner GbR**
**Patent- und Rechtsanwälte**
**Theresienhöhe 13**
**80339 München (DE)**

(54) **COMPOSITION CONTAINING BEE VENOM AS AN ACTIVE INGREDIENT FOR PREVENTING AND TREATING ACNE**

(57) The present invention relates to a composition for preventing and treating acne, comprising a material which has excellent antibacterial activity against *Propionibacterium acnes* known as acne-causing bacteria and which shows strong antibacterial activity against skin floras that enlarge the site of inflammation to worsen acne, in which the material also helps to regenerate skin cells damaged by acne. The composition comprises bee venom (collected from *Apis meliffera*) and is used the form of soap, cosmetic products, and medical products for skin application such as ointments, bands and dressings, which are used to fundamentally prevent and treat acne.

[Fig. 1]

EP 2 460 526 A2

## Description

## Technical Field

The present invention relates, in general, to compositions for preventing and treating acne, comprising bee venom as an active ingredient, and more particularly to pharmaceutical and cosmetic compositions for preventing and treating acne, comprising bee venom which has excellent antibacterial activity against *Propionibacteribacterium acnes* known as acne-causing bacteria and anaerobic skin floras that enlarge the site of inflammation caused by acne to worsen acne, in which the bee venom also has an excellent ability to regenerate damaged skin cells.

## Background Art

The exact cause of acne is unknown, but doctors believe that it results from major interrelated factors, including increased sebaceous secretion, the progression of inflammation by the male hormone androgen stimulating sebaceous glands and by *Propionibacterium acnes* that propagates in hair sebaceous glands to degrade sebum to form free fatty acids, follicular parakeratosis, and genetic predisposition. Also, aerobic skin flora is not the primary cause of inflammation, but is known to enlarge the site of inflammation, thus contributing to worsening acne. General methods which are known to be used for treatment of acne include administration of oral antibiotics and retinoid, application of external-use drugs, comedo extraction, chemical peeling, etc. Particularly, inflammatory acne resulting from bacterial infection can be relieved by treatment with antibiotics, but it has recently been reported that antibiotics, such as tetracycline, clindamycin and erythromycin, which are mainly used to inhibit the proliferation of P. *acnes* and reduce inflammatory reactions, can increase P. *acnes* having resistance to antibiotics and cause side effects. Until now, in order to treat acne, in the medical field, antibiotics such as erythromycin or benzoyl peroxide have been used to inhibit P. *acnes,* or the female hormone estrogen has been used to control sebum, but these drugs cause side effects. In the cosmetic field, vitamin A derivatives have been used to remove skin keratin, and triclosan and salicylic acid have been used to inhibit P. *acnes.* These drugs show some effects, but mostly cause side effects, such as skin erythema, skin hypersensitivity reactions or photosensitivity reactions, and acne frequently recurs after treatment with these drugs. Thus, there is no pharmaceutical product for the prevention and treatment of acne.

Bee venom of *Apis mellifera,* which is produced in Korea, can be collected in large amounts by a bee venom collector using an electric shock method without causing damage to bees, and purified bee venom can be obtained by removing foreign matter from the collected bee venom, followed by freeze drying. The bee venom thus obtained contains various components, and among these components, peptides have anti-inflammatory and antibacterial effects, potent pain-killing effects, immune-enhancing effects, etc. Bee venom shows antibacterial activity against both gram-positive and gram-positive bacteria, but is known to show stronger antibacterial activity against gram-positive bacteria. Most of the pharmaceutical effects of bee venom are attributable to the activities of venomous components and single major component groups. The known components and effects of bee venom are as follows.

(1) Treatment of immune system diseases: Bee venom is effective in the treatment of most immune diseases and can stimulate the immune system to successfully fight diseases. The two major immune functions of bee venom are to stimulate the biological system of organisms and to enhance the defensive power of the body.

(2) Anti-inflammatory effect: The main components of bee venom are very effective in the inhibition of inflammation. Particularly, when melittin and apamin were injected into arthritis patients, edema was significantly inhibited. Also, treatment with a protease inhibitor and dolapin inhibited edema by 15-40 %.

(3) Neurotoxic effect: Getting stung by a bee causes pain and inflammation, and the bee venom components that cause pain and inflammation are effectively used in the development of pain-killing agents for pain withdrawal responses in mammals. Particularly, apamin in bee venom acts to kill pain and shows strong pain-killing effects and high therapeutic effects in neuralgia, arthritis, rheumatoid arthritis, gout, muscular pain, etc.

(4) Hemolytic effect: one of the most excellent effects of bee venom is a hemolytic effect. Melittin, phospholipase and like in bee venom show therapeutic and hemolytic effects by absorbing and excreting extravasated blood from contrusions or internal hemorrhage supplying fresh blood, oxygen and nutrients to tissue.

(5) Vasodilating effect: Histaminic substances in bee venom are greatly effective in enlarging capillary vessels, arterioles, veinlets, and particularly visceral vessels. Thus, bee venom is used in the treatment of diseases such as sensitivity of cold, frostbite and muscular pain.

(6) Blood pressure-lowering effect: Histaminic substances in bee venom have a potent effect of lowering blood pressure. Particularly, they show blood pressure-lowering action even at a concentration of $1/2.5 \times 10^7$ and are highly effective in the treatment of essential hypertension and the like.

(7) Autonomic nerve-regulating effect: When the human body is continuously stressed, autonomic nerves in the sympathetic and parasympathetic nerve systems become irregular, thus causing many diseases. Catecholamine and acetylcholine, which are required to normalize the autonomic nerve, are contained in bee venom, and these components are brain cell transmitters that are effective in the treatment of psychosomatic disease, menopausal disorder, stress-induced diseases, etc.

(8) Antibacterial effect: Bee venom has very excellent antibacterial and antifungal effects and is also effective against viral tumors. Particularly, it is highly effective in the treatment of periodontitis, tonsillitis, sties, suppurative diseases, etc.

Based on the above-described physiological effects of bee venom, the present inventors have conducted studies to provide a bee venom-based composition effective in the prevention and treatment of acne. According to the present invention, the quality of life of persons suffering from severe acne will be improved, and the consumption of bee venom which is produced in Korea will be promoted while the global position of Korean bee venom will be raised.

## Disclosure

### Technical Problem

The present invention relates to a composition for preventing and treating acnes. More specifically, the present invention provides a composition for preventing and treating acne comprising bee venom which has an excellent antibacterial effect against *Propionibacterium acnes* known to worsen acne and has an excellent anti-inflammatory effect of inhibiting or relieving inflammation caused by various skin troubles such as acne, in which the composition can be used in the form of soap, cosmetic products, and medical products such as ointments, bands and dressings.

Accordingly, the present inventors have made extensive efforts to develop cosmetic and medical products effective for the prevention and treatment of acne, and as a result, have developed a composition effective for the prevention and treatment of acne, which comprises, as an active ingredient, bee venom expected to have excellent antibacterial, anti-inflammatory and cell-regenerating effects.

### Technical Solution

One embodiment of the present invention provides a composition for preventing or treating acne, which comprises bee venom as an active ingredient.

Another embodiment of the present invention provides a cosmetic composition for preventing or treating acne, which comprises bee venom as an active ingredient.

Still another embodiment of the present invention provides a body cleansing or cleaning product for preventing or treating acne, which comprises bee venom as an active ingredient.

The concentration of bee venom in the concentration may be 0.0001-10% (w/v).

Specifically, the present invention provides a composition for preventing and treating acne, which comprises purified bee venom produced in Korea.

The content of purified bee venom in the composition for preventing and treating acne according to the present invention is 0.0001-10% (w/v), and preferably 0.01-1% (w/v). If the content of bee venom in the composition is less than 0.0001%, the antibacterial and anti-inflammatory effects of the composition against P. *acnes* will be low, and if the content is more than 10%, the composition will be cytotoxic.

Meanwhile, a composition for skin application comprising bee venom may be present in any state such as a liquid, cream, paste or solid state and may be formulated in the form of cream, skin lotion, pack, makeup base, facial cleansers and cleaners such as soap, facial foam or body cleanser, and products for hair such as shampoo or rinse. If necessary, the composition may be formulated together with components that are generally used in compositions for skin application, for example, aqueous components, oily components, surfactants, moisturizers, thickeners, preservatives, fragrances, pigments, etc. Also, the composition may be mixed with a pharmaceutically acceptable carrier, forming agent or diluent and formulated in the form of powders, granules, capsules or injectable solutions. In addition, the composition may also be applied as a body cleansing or cleaning products such as wet tissue.

Further, the composition of the present invention may be used in combination of antibacterial agents, such as benzoyl peroxide, tetracycline, erythromycin or triclosan, hormones such as androgen agents or steroids, or vitamin A derivatives such as retinoid, which have been used in existing acne-treating agents.

### Advantageous Effects

As described above, purified bee venom obtained by removing foreign matter from the domestically produced bee venom collected from bees using a bee venom collector has excellent antibacterial effects against *Propionibacterium acnes,* clindamycin-resistant P. *acnes,* and *Staphylococcus aureus, Staphylococcus epidermidis* and Sreptococcus pyogenes, which are known to worsen acne by enlarging the sites of inflammation. Also, the bee venom can rapidly and efficiently regenerate damaged skin cells and can promote the regeneration of skin cells such that damaged sites are not inflamed. In addition, the bee venom promotes the synthesis of collagen and the expression of EGF and VEGF which are skin cell growth factors and has excellent effects on the prevention of wrinkles and aging. Thus, the present invention can provide cosmetic products, beauty soaps, and medical products such as ointments, bands or dressings, which comprise bee venom and are used for the prevention and treatment of acne. Particularly, the present invention provides lotion, nourishing lotion, nourishing cream, massage cream, and essence, which comprise bee venom.

Accordingly, the composition for preventing and treating acne comprising bee venom, developed according to the present invention, will greatly contribute to the treatment of patients suffering from severe acne and will contribute to increasing the beekeeper's income due to the high-value-added use of domestically produced bee venom.

## Description of Drawings

FIG. 1 shows the results of evaluating the cytotoxicity of bee venom against HEK (human epidermal keratinocyte) cells.
FIG. 2 shows the effect of bee venom on the regeneration of damaged HEK (human epidermal keratinocyte) cells.
FIG. 3 shows collagen synthesis after treatment of damaged HEK (human epidermal keratinocyte) cells with bee venom.
FIG. 4 shows EGF production after treatment of damaged HEK (human epidermal keratinocyte) cells with bee venom.
FIG. 5 shows VEGF production after treatment of damaged HEK (human epidermal keratinocyte) cells with bee venom.

## Mode for Invention

Hereinafter, the present invention will be described in further detail with reference to examples. However, the scope of the present invention is not limited to these examples.

All data were subjected to the analysis of variance using Duncan's t-test of SAS (SAS enterprise guide 3.0).

Example 1: Separation of effective components from bee venom

Bee venom was collected from *Apis meliffera* L. using a bee venom collector. The collected bee venom was dissolved in distilled water, centrifuged, filtered and freeze-dried, thereby obtaining pure bee venom which was used in the test.

Example 2: Measurement of antibacterial activity

Test Example 1: Measurement of antibacterial activity against acne-causing bacteria

Antibacterial effects against P. *acnes* known to cause acne and antibiotic clindamycin-resistant P. *acnes* were measured. Minimum inhibitory concentrations (MICs) against P. *acnes* and antibiotic clindamycin-resistant P. *acnes* were measured using a broth dilution method.

Dried bee venom was diluted with distilled water, aseptically filtered and serially diluted according to a broth dilution method. Each of P. *acnes* and antibiotic-resistant P. *acnes* was anaerobically cultured in a TS (trypticase soy) broth supplemented with 5% sheep blood 35 C for 48 hours and was then used in the test. Each of the bacterial strains was added to a well plate at a cell density of $2 \times 10^6$ CFU/well and cultured together with a bee venom sample for 24 hours, after which the growth of the bacterial strains was visually and microscopically observed and the absorbance at an absorption wavelength of 540 nm was measured. The sample concentration showing the same absorbance value as that of the pure broth was determined as the minimum inhibitory concentration (MIC). The results of the measurement are shown in Table 1 below.

[Table 1]

| Bacterial strains | MIC ($\mu$g/m$\ell$) |
|---|---|
| *Propionibacterium acnes* (ATCC 6919) | 0.086±0.013 |

(continued)

| Bacterial strains | MIC ($\mu$g/m$\ell$) |
|---|---|
| Antibiotic-resistant *P. acnes* (CCARM 9010) | 0.067±0.028 |
| * mean ± SD | |

Test Example 2: Measurement of antibacterial activities against anaerobic skin floras

*Staphylococcus aureus, Staphylococcus epidermidis* and *Sreptococcus pyogenes,* which are typical anaerobic skin floras, were cultured in a Muller-Hiton broth at 37 °C for 18 hours and then used in the test, and *S. pyogenes* was cultured in a $CO_2$ incubator. Each of the bacterial strains was added to a well plate at a cell density of $2\times10^6$ CFU/well and cultured together with a bee venom sample for 24 hours, after which the growth of the bacterial strains was visually and microscopically observed and the absorbance at an absorption wavelength of 540 nm was measured. The sample concentration showing the same absorbance value as that of the pure broth was determined as the minimum inhibitory concentration (MIC). The results of the measurement are shown in Table 2 below.

[Table 2]

| Bacterial strains | MIC ($\mu$g/m$\ell$) |
|---|---|
| *Staphylococcus aureus* (ATCC 25923) | 0.071±0.042 |
| *Staphylococcus epidermidis* (ATCC 12228) | 0.121±0.028 |
| *Sreptococcus pyogenes* (ATCC 12353) | 0.104±0.049 |
| * mean ± SD | |

Test Example 3: Measurement of postantibiotic effects of bee venom against acne-causing bacteria

The postantibiotic effects (PAEs) of the bee venom, obtained in Example 1, against the acne-causing strain *P. acnes* and the antibiotic clindamycin-resistant *P. acnes* were measured according to the method of Pankuch and Appelbaum (2006). Briefly speaking, each of the cultured bacterial strains was treated with bee venom at the concentration described in Example, and after 1 hour, the bee venom was removed by centrifugation and a broth dilution method. Then, the broth was replaced with a fresh broth and cultured in an incubator at 37 °C while the number of bacterial cells was measured at 2-hr intervals. Higher PAE values indicate higher antibacterial effects.

PAE was calculated according to the following equation:

$$[\text{PAE} = \text{T-C}]$$

wherein T: the time taken for growth to 1log10 in a test group treated with a natural antibiotic; and C: the time taken for growth to 1log10 in a non-treated group.

The results of the measurement are shown in Table 3 below.

[Table 3]

| Bacterial strains | PAE (hrs) |
|---|---|
| *Propionibacterium acnes* (ATCC 6919) | 4.30 |
| Antibiotic-resistant *P. acnes* (CCARM 9010) | 5.45 |
| * Number of inoculated bacterial cells: 2.5± $10^5$ CFU/m$\ell$ | |

Test Example 4: Measurement of postantibiotic effects of bee venom against skin floras

A test was performed in the same manner as Test Example 3, except that *Staphylococcus aureus, Staphylococcus epidermidis* and *Sreptococcus pyogenes* were used. The results of the test are shown in Table 4 below.

[Table 4]

| Bacterial strains | MIC (μg/mℓ) |
|---|---|
| *Staphylococcus aureus* (ATCC 25923) | 0.071±0.042 |
| *Staphylococcus epidermidis* (ATCC 12228) | 0.121±0.028 |
| *Sreptococcus pyogenes* (ATCC 12353) | 0.104±0.049 |
| * Number of inoculated bacterial cells: 2.5± $10^5$ CFU/mℓ | |

Example 3: Measurement of effect on regeneration of skin cells

Test Example 5: Measurement of skin cell cytoxicity

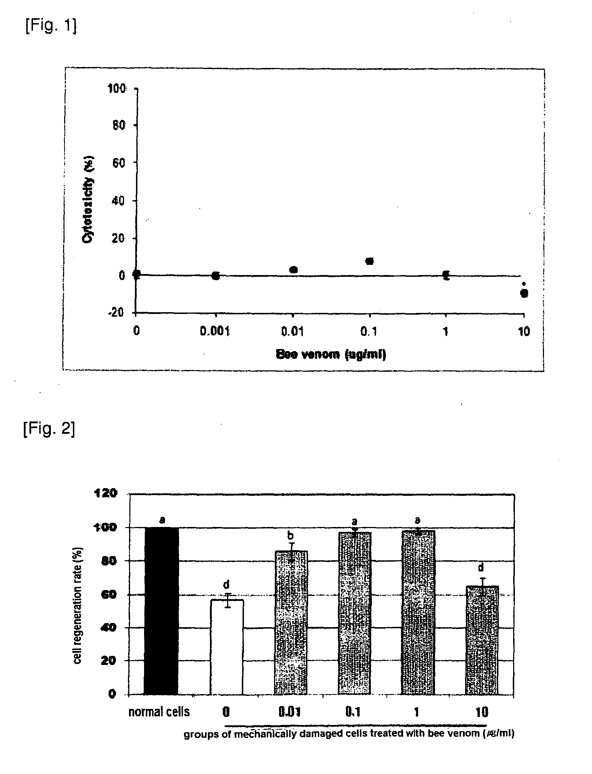

In order to examine the cytotoxicity of bee venom against skin cells, primarily cultured HEK (human epidermal keratinocyte) cells were purchased from MCTT Co. (Seoul, Korea) and subcultured in KGM medium in a 5% $CO_2$ incubator at 37 °C. As the cultured cells were grown to a confluence of 90%, they were subcultured, and cells in the log-growth phase were used in the test. HEK cells were dispensed in a 96-well plate at a cell density of $5×10^5$ cell/ml and cultured adherently for one day, and the medium was replaced with a fresh medium, after which the cells were treated with various concentration of bee venom. After the cells have been cultured in a 5% $CO_2$ incubator at 37 °C for 48 hours, the cells were treated with MTT (methylthiazol-2-yl-2.5-diphenyl tetrazolium bromide) reagent, and the absorbance at 540 nm was measured with an ELISA reader, thereby evaluating the skin cell toxicity of bee venom.
The results of the evaluation are shown in FIG. 1.

Test Example 6: Test for cell regeneration and growth factor expression in skin wound model

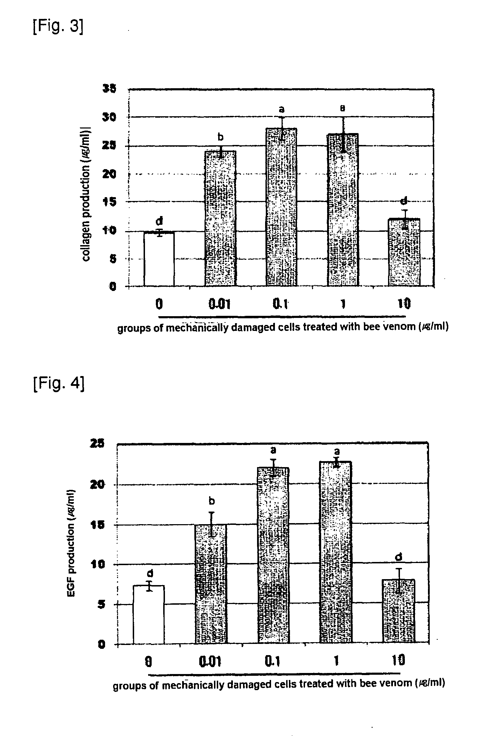

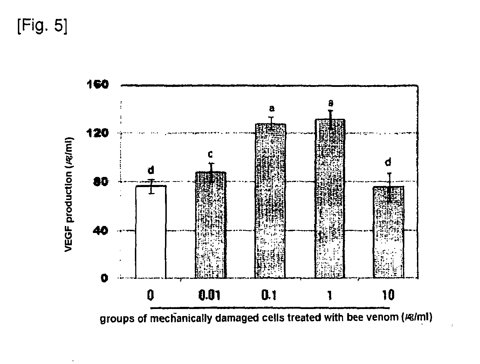

When primarily cultured HEK (Human Epidermal Keratinocyte) cells were grown to a confluence of 80% in a 100-mm cell culture dish, the cells were wounded with a toothpick to make a wound having a width of 10 mm, and 0.01-10 μg/mℓ of bee venom was injected into the cells, after which the regeneration of the cells was measured.
Herein, the proliferation of the cells was measured by analyzing the cells with MTT (methylthiazole-2-yl-2.5-diphenyl tetrazolium bromide) or by staining the cells with Diff quic stain and observing the cells with a microscope. The synthesis of collagen was analyzed using a collagen assay kit, and the expression of epidermal growth factor (EGF) and a vascular endothelial growth factor (VEGF), which are involved in cell growth, was analyzed using an ELISA (enzyme-linked immunosorbent assay) kit. The results of the analysis are shown in FIGS. 2 to 5.
As can be seen in FIG. 2, treatment of the cells with bee venom promoted the regeneration of damaged skin cells, and as can be seen in FIG. 3, treatment of the cells with bee venom increased the production of collagen. Also, as can be seen in FIGS. 4 and 5, when the cells were treated with bee venom, the productions of the skin cell growth factors EGF and VEGF were increased by 2 times or more, respectively. Meanwhile, the morphology of the grown cells was normal.

Example 4: Preparation of cosmetic products comprising bee venom

Preparation Example 1: Formulation of lotion comprising bee venom

[Table 5]

| No. | Raw material | Formulation 1 (wt%) | Comparative formulation 1 (wt%) |
|---|---|---|---|
| 1 | Purified bee venom | 0.001 | - |
| 2 | Glycerin | 3.0 | 3.0 |
| 3 | Butylene glycol | 2.0 | 2.0 |
| 4 | Propylene glycol | 2.0 | 2.0 |
| 5 | polyoxytethylene (60) hydrogenated castor oil | 1.0 | 1.0 |
| 6 | Ethanol | 10.0 | 10.0 |

(continued)

| No. | Raw material | Formulation 1 (wt%) | Comparative formulation 1 (wt%) |
|---|---|---|---|
| 7 | Triethanolamine | 0.1 | 0.1 |
| 8 | Preservative | Trace | Trace |
| 9 | Pigment | Trace | Trace |
| 10 | Fragrance | Trace | Trace |
| 11 | Purified water | Balance | Balance |

Formulation 1: Raw materials 2, 3, 4 and 8 were sequentially added to raw material 11, and the mixture was dissolved with stirring. Then, raw material 5 was dissolved by heating to about 60 °C, and then raw material 10 was added thereto and stirred, and the mixture was added to raw material 11. Finally, raw materials 1, 6, 7 and 9 were added thereto and sufficiently stirred, and the mixture was passed through a high-pressure homogenizer and then aged.
Comparative formulation 1: Comparative formulation 1 was prepared in the same manner as Formulation 1, except that bee venom (raw material 1) was not used.

Preparation Example 2: Formulation of nourishing lotion comprising bee venom

[Table 6]

| No. | Raw material | Formulation 1 (wt%) | Comparative formulation 1 (wt%} |
|---|---|---|---|
| 1 | Purified bee venom | 0.001 | - |
| 2 | Sitosterol | 1.7 | 1.7 |
| 3 | Polyglycein 2-oleate | 1.5 | 1.5 |
| 4 | Ceramide | 0.7 | 0.7 |
| 5 | Stearate-4 | 1.2 | 1.2 |
| 6 | Cholesterol | 1.5 | 1.5 |
| 7 | Dicetyl phosphate | 0.4 | 0.4 |
| 8 | Glycerin | 5.0 | 5.0 |
| 9 | Sunflower oil | 10.0 | 10.0 |
| 10 | Carboxyvinyl polymer | 0.2 | 02 |
| 11 | Xanthan gum | 0.3 | 0.3 |
| 12 | Preservative | Trace | Trace |
| 13 | Fragrance | Trace | Trace |
| 14 | Purified water | Balance | Balance |

Formulation 1: Raw materials 2, 3, 4, 5 and 6 were homogenized at a predetermined temperature to prepare a nonionic amphiphatic lipid. The amphiphatic lipid was mixed with raw materials 1, 7, 8 and 14 and homogenized at a predetermined temperature, and the mixture was passed through a high-pressure homogenizer. Then, raw material 9 was added slowly thereto at a predetermined temperature and homogenized, and the mixture was passed through a high-pressure homogenizer. Then, raw materials 10, 11, 12 and 13 were added thereto, dispersed, stabilized and aged.
Comparative formulation 1: Comparative formulation 1 was prepared in the same manner as Formulation 1, except that bee venom (raw material 1) was not used.

Preparation Example 3: Formulation of nourishing cream comprising bee venom

[Table 7]

| No. | Raw material | Formulation 1 (wt%) | Comparative formulation 1 (wt%) |
|-----|--------------|---------------------|--------------------------------|
| 1 | Purified bee venom | 0.001 | - |
| 2 | Sitosterol | 4.0 | 4.0 |
| 3 | Polyglycerin 2-oleate | 3.0 | 3.0 |
| 4 | Ceramide | 0.7 | 0.7 |
| 5 | Stearate-4 | 2.0 | 2.0 |
| 6 | Cholesterol | 3.0 | 3.0 |
| 7 | Dicetyl phosphate | 0.4 | 0.4 |
| 8 | Glycerin | 5.0 | 5.0 |
| 9 | Sunflower oil | 22.0 | 22.0 |
| 10 | Carboxyvinyl polymer | 0.5 | 0.5 |
| 11 | Xanhan gum | 0.5 | 0.5 |
| 12 | Triethanolamine | Trace | Trace |
| 13 | Fragrance | Trace | Trace |
| 14 | Purified water | Balance | Balance |

Formulation 1: Raw materials 2, 3, 4, 5 and 6 were homogenized at a predetermined temperature to prepare a nonionic amphiphatic lipid. The amphiphatic lipid was mixed with raw materials 1, 7, 8 and 14 and homogenized at a predetermined temperature, and the mixture was passed through a high-pressure homogenizer. Then, raw material 9 was added slowly thereto at a predetermined temperature and homogenized, and the mixture was passed through a high-pressure homogenizer. Then, raw materials 10, 11, 12 and 13 were added thereto, dispersed, stabilized and aged.
Comparative formulation 1: Comparative formulation 1 was prepared in the same manner as Formulation 1, except that bee venom (raw material 1) was not used.

Preparation Example 4: Formulation of massage cream comprising bee venom

[Table 8]

| No. | Raw material | Formulation 1(wt%) | Comparative Formulation 1(wt%) |
|-----|--------------|--------------------|-------------------------------|
| 1 | Purified bee venom | 0.001 | - |
| 2 | Beeswax | 10.0 | 10.0 |
| 3 | Polysorbate 60 | 1.5 | 1.5 |
| 4 | Sorbitan sesquioleate | 0.8 | 0.8 |
| 5 | Liquid paraffin | 40.0 | 40.0 |
| 6 | Squalene | 5.0 | 5.0 |
| 7 | Caprylic/capric triglyceride | 4.0 | 4.0 |
| 8 | Carboxvinyl polymer | 0.2 | 0.2 |
| 9 | Glycerin | 5.0 | 5.0 |
| 10 | Butylene glycol | 3.0 | 3.0 |
| 11 | Propylene glycol | 3.0 | 3.0 |
| 12 | Triethanolamine | 0.2 | 0.2 |

(continued)

| No. | Raw material | Formulation 1(wt%) | Comparative Formulation 1(wt%) |
|-----|--------------|--------------------|--------------------------------|
| 13 | Preservative | Trace | Trace |
| 14 | Pigment | Trace | Trace |
| 15 | Fragrance | Trace | Trace |
| 16 | Purified water | Balance | Balance |

Formulation 1: Raw materials 8, 9, 10, 11, 13 and 16 were mixed with stirring while they were heated to a temperature between 80 °C and 85 °C, and then introduced into a preparation unit. Then, raw materials 2, 3, 4, 5, 6, 7 and 12 were heated to a temperature between 80 °C and 85 °C and introduced into the preparation unit, and the mixture was emulsified. After completion of emulsification, the emulsified mixture was stirred while it was cooled to 50 °C. Then, raw material 14 was added thereto, and the mixture was cooled to 45 °C. Then, raw material 15 was added thereto and the mixture was cooed to 35 °C, and raw material 1 was added thereto. The resulting mixture was cooled to 25 °C and then aged.

Comparative formulation 1: Comparative formulation 1 was prepared in the same manner as Formulation 1, except that bee venom (raw material 1) was not used.

Preparation Example 5: Formulation of essence comprising bee venom

[Table 9]

| No. | Raw material | Formulation 1(wt%) | Comparative Formulation 1 (wt%) |
|-----|--------------|--------------------|--------------------------------|
| 1 | Purified bee venom | 0.001 | - |
| 2 | Sitosterol | 1.7 | 1.7 |
| 3 | Polyglycerin 2-oleate | 1.5 | 1.5 |
| 4 | Ceramide | 0.7 | 0.7 |
| 5 | Ceramide | 1.2 | 1.2 |
| 6 | Stearate-4 | 1.5 | 1.5 |
| 7 | Dicetyl phosphate | 0.4 | 0.4 |
| 8 | Glycerin | 5.0 | 5.0 |
| 9 | Sunflower oil | 15.0 | 15.0 |
| 10 | Carboxyvinyl polymer | 0.2 | 0.2 |
| 11 | Xanthan gum | 0.2 | 0.2 |
| 12 | Preservative | Trace | Trace |
| 13 | Fragrance | Trace | Trace |
| 14 | Purified water | Balance | balance |

Formulation 1: Raw materials 2, 3, 4, 5 and 6 were homogenized at a predetermined temperature to prepare a nonionic amphiphatic lipid. The amphiphatic lipid was mixed with raw materials 1, 7, 8 and 14 and homogenized at a predetermined temperature, and the mixture was passed through a high-pressure homogenizer. Then, raw material 9 was added slowly thereto at a predetermined temperature and homogenized, and the mixture was passed through a high-pressure homogenizer. Then, raw materials 10, 11, 12 and 13 were added thereto, dispersed, stabilized and aged.

Comparative formulation 1: Comparative formulation 1 was prepared in the same manner as Formulation 1, except that bee venom (raw material 1) was not used.

**Claims**

1. A composition for preventing or treating acne, which comprises bee venom as an active ingredient.

2. The composition of claim 1, wherein a concentration of the bee venom in the composition is 0.0001-10% (w/v).

3. A cosmetic product for preventing or treating acne, which comprises bee venom as an active ingredient.

4. The cosmetic product of claim 3, wherein a concentration of the bee venom in the cosmetic product is 0.0001-10% (w/v).

5. A body cleansing or cleaning product for preventing or treating acne, which comprises bee venom as an active ingredient.

6. The body cleansing or cleaning product of claim 5, wherein a concentration of the bee venom in the product is 0.0001-10% (w/v).

[Fig. 1]

[Fig. 2]

[Fig. 3]

groups of mechanically damaged cells treated with bee venom (㎍/ml)

[Fig. 4]

groups of mechanically damaged cells treated with bee venom (㎍/ml)

[Fig. 5]

groups of mechanically damaged cells treated with bee venom (µg/ml)